# EUROPÄISCHE PATENTANMELDUNG

(11) **EP 3 345 890 A1**
(43) Veröffentlichungstag der Anmeldung: **11.07.2018**
(21) Anmeldenummer: 17150428.5
(22) Anmeldetag: 05.01.2017
(51) Int. Cl.: C07C 209/18, C07C 211/48, B01J 23/72, B01J 23/06, B01J 21/02, C10L 1/223

(54) **VERFAHREN ZUR HERSTELLUNG VON N-METHYL-P-TOLUIDIN FÜR DESSEN EINSATZ ALS ADDITIV FÜR FLUGBENZIN**

(71) Anmelder: Lanxess Deutschland GmbH, 50569 Köln (DE)
(72) Erfinder: Die Erfindernennung liegt noch nicht vor

(57) **Zusammenfassung**

Die Erfindung betrifft neue Verfahren zur Herstellung von N-Methyl-p-toluidin für dessen Einsatz als Additiv für Flugbenzin sowie spezielle Katalysatoren für diese Verfahren.

## Beschreibung

Die Erfindung betrifft neue Verfahren zur Herstellung von N-Methyl-p-toluidin für dessen Einsatz als Additiv für Flugbenzin sowie spezielle Katalysatoren für diese Verfahren.

Alkylierte aromatische Amine haben ein hohes wirtschaftliches Potential und werden beispielsweise als Treibstoffadditive (Antiklopfmittel) in Benzinen eingesetzt, um umweltschädliche, bleihaltige Additive abzulösen. Das am häufigsten eingesetzte alkylierte aromatische Amin ist N-Methylanilin. Daneben können aber auch N-alkylierte m- und p-Toluidine zum Einsatz kommen. Für die Herstellung von N-methylierten Toluidinen und speziell N-Methyl-p-toluidin sind nur wenige wirtschaftlich relevante Verfahren beschrieben. US-A5030759 und DE-A3803661 beschreiben die N-Alkylierung von Anilin und Toluidinen an Zeolithkatalysatoren. US-A2580284 beschreibt die N-Alkylierung von Anilin an aluminiumoxidbasierten Trägerkatalysatoren, die als katalytisch aktives Element Kupfer und Promotoren wie Zinkoxid oder Calciumoxid enthalten. Aus DE-A3942413 ist die Verwendung von reiner Niobsäure oder Niobsäure im Gemisch mit inerten Feststoffen wie Titanoxid, Zinkoxid etc. bekannt, was aber zu relativ hohen Anteilen dialkylierter Produkte führt. DE-A3840194 beschreibt die Verwendung von auf Aluminiumoxid geträgerten Kupfer-Katalysatoren, die mit Mangan bzw. Seltenerdverbindungen imprägniert sind. Hauptproblem in den oben geschilderten Beispielen ist die kurze Standzeit der bislang eingesetzten Katalysatoren, die durch Nebenproduktbildungen auf der Katalysatoroberfläche hervorgerufen wird. So berichtet RU-2205067 für die N-Methylierung von Anilin bei Verwendung eines Cu/Mn/Cr/Fe/Co-Trägerkatalysators auf Aluminiumoxid eine max. Standzeit von 230 Stunden. In RU-2274488 werden mit ähnlichen Systemen Laufzeiten bis max. 470 Stunden berichtet. Alternativ dazu ist aus RU-2270187 sowie RU-2223258 die Herstellung von N-Methylanilin über regenerierende N-Methylierung des Anilins mit Methanol sowie durch katalytische Hydrierung des Nitrobenzols mit Wasserstoff und nachfolgender Alkylierung mit Methanol an CuO/Cr₂O₃/Al₂O₃-Katalysatoren bekannt. Die Verfahren sind jedoch alle ökonomisch und/oder ökologisch verbesserungsbedürftig, da einerseits nur geringe Durchsätze erreicht werden und andererseits viele Katalysatoren kanzerogene Komponenten enthalten.

Aufgabe der vorliegenden Erfindung war daher die Bereitstellung eines Verfahrens zur Herstellung von N-Methyl-p-toluidin in hohen Ausbeuten, einer langen Standzeit des Katalysators bei hohem Durchsatz und Vermeidung kanzerogener Metalle bzw. deren Oxiden und idealerweise die Bereitstellung eines neuen, wirtschaftlich herstellbaren Katalysators.

Überraschenderweise wurde nun gefunden, dass die erfindungsgemäße Aufgabe dadurch gelöst werden kann, dass mindestens ein Katalysator eingesetzt wird, der zumindest in folgenden Anteilen
Cu: 45,5 - 68,0 Gew.%, vorzugsweise 47,0 - 64,1 Gew.%, besonders bevorzugt 47,5 - 60,5 Gew.%,
Zn: 12,3 - 22,5 Gew.%, vorzugsweise 14,3 - 21,5 Gew.%, besonders bevorzugt 15,2 - 19,5 Gew.%,
Al: 1,9 - 4,1 Gew.%, vorzugsweise 2,2 - 3,8 Gew.%, besonders bevorzugt 2,5 - 3,6 Gew.%,
sowie 7,8 - 28,2 Gew.% Sauerstoff, vorzugsweise 13,6 - 25,1 Gew.%, besonders bevorzugt 16,2 - 24,5 Gew.% Sauerstoff und gegebenenfalls 0 - 10 Gew.% Kohlenstoff enthält, wobei Cu, Zn, Al, Sauerstoff und gegebenenfalls Kohlenstoff mindestens 99 Gew.%, vorzugsweise 99,5 Gew.% der Gesamtmenge des Katalysators umfassen.

Gegenstand der vorliegenden Erfindung sind daher Verfahren zur Herstellung von N-Methyl-p-toluidin durch Umsetzung von p-Toluidin mit Methanol, wonach mindestens ein Katalysator eingesetzt wird, der zumindest in folgenden Anteilen
Cu: 45,5 - 68,0 Gew.%, vorzugsweise 47,0 - 64,1 Gew.%, besonders bevorzugt 47,5 - 60,5 Gew.%,
Zn: 12,3 - 22,5 Gew.%, vorzugsweise 14,3 - 21,5 Gew.%, besonders bevorzugt 15,2 - 19,5 Gew.%,
Al: 1,9 - 4,1 Gew.%, vorzugsweise 2,2 - 3,8 Gew.%, besonders bevorzugt 2,5 - 3,6 Gew.%,
sowie 7,8 - 28,2 Gew.% Sauerstoff, vorzugsweise 13,6 - 25,1 Gew.%, besonders bevorzugt 16,2 - 24,5 Gew.% Sauerstoff und gegebenenfalls 0 - 10 Gew.% Kohlenstoff enthält, wobei Cu, Zn, Al, Sauerstoff und gegebenenfalls Kohlenstoff mindestens 99 Gew.%, vorzugsweise 99,5 Gew.% der Gesamtmenge des Katalysators umfassen.

Andere Bestandteile des Katalysators können beispielsweise Alkali- und Erdalkalimetalle, Wasser oder Stickstoffverbindungen, wie beispielsweise Nitrate sein.

In einer bevorzugten Ausführungsform enthält der Katalysator zumindest in folgenden Anteilen
kupferoxidhaltige Verbindungen, gerechnet als CuO 59,1 - 78,3 Gew.%, besonders bevorzugt 62,3 - 74,8 Gew.%,
ZnO: 15,3 - 27,8 Gew.%, besonders bevorzugt 17,5 - 25,2 Gew.%,
Al₂O₃: 4,4 - 6,8 Gew.%, besonders bevorzugt 4,9 - 6,1 Gew.%,
sowie 0 - 10 Gew.% Kohlenstoff, wobei CuO, ZnO, Al₂O₃ und gegebenenfalls Kohlenstoff mindestens 99 Gew.%, vorzugsweise 99,5 Gew.% der Gesamtmenge des Katalysators umfassen.

In einer Ausführungsform der Erfindung wird der Katalysator durch Zufuhr von Wasserstoff, vorzugsweise in Gegenwart eines Inertgases, bei Temperaturen von mindestens 150 °C vorbehandelt.

Die Vorbehandlung des Katalysators mit Wasserstoff kann vor dem Einsatz in den Reaktor oder im Reaktor selber, d. h. im Reaktor, der für die Herstellung von N-Methyl-p-toluidin genutzt wird, erfolgen.

Eine Vorbehandlung des Katalysators erfolgt vorzugsweise dadurch, dass dieser in eine Reduktionsapparatur eingefüllt und darin auf vorzugsweise 150 - 300 °C, besonders bevorzugt 200 - 300 °C und ganz besonders bevorzugt auf 240 - 290 °C aufgeheizt wird. Vorzugsweise wird der Katalysator mit einem Stickstoff/Wasserstoffstrom einer anfänglichen Zusammensetzung von 95 % N₂ und 5 % H₂ beaufschlagt. Vorzugsweise wird der Wasserstoffanteil im Gasstrom auf 100 % gesteigert. Die vorgenannte Vorbehandlung hat in der Regel zur Folge, dass CuO zu elementarem Cu reduziert wird, während die anderen Metalle als Oxide vorliegen.

Für die Vorbehandlung des Katalysators mit Wasserstoff im Reaktor selber wird dieser im vorgenannten Reaktor unter einem Stickstoff/Wasserstoffstrom vorzugsweise der anfänglichen Zusammensetzung 95 % N₂ und 5 % H₂ auf vorzugsweise 150 - 300 °C, bevorzugt 200 - 300 °C und besonders bevorzugt auf 240 - 290 °C aufgeheizt und dann vorzugsweise der Wasserstoffanteil im Gasstrom auf 100 % H₂ gesteigert.

In einer bevorzugten Ausführungsform des erfindungsgemäßen Verfahrens liegt die Belastung des Katalysators bei 0,1 bis 1,5, vorzugsweise 0,3 bis 1, besonders bevorzugt 0,4 bis 0,7 kg p-Toluidin pro kg Katalysator und Stunde.

In dem erfindungsgemäßen Verfahren zur Herstellung von N-Methyl-p-toluidin kann dabei p-Toluidin in handelsüblicher Qualität z. B. von der Lanxess Deutschland GmbH eingesetzt werden.

In dem erfindungsgemäßen Verfahren zur Herstellung von N-Methyl-p-toluidin kann dabei Methanol in handelsüblicher Qualität z. B. von der Brenntag GmbH eingesetzt werden.

In dem erfindungsgemäßen Verfahren läuft die Reaktion vorzugsweise in einem Rohrreaktor ab.

In einer weiteren bevorzugten Ausführungsform des erfindungsgemäßen Verfahrens beträgt das molare Verhältnis von p-Toluidin zu Methanol 1:0,7 bis 1:5, vorzugsweise 1:1 bis 1:3,5, besonders bevorzugt 1:1,5 bis 1:2,5.

In einer ebenfalls bevorzugten Ausführungsform des erfindungsgemäßen Verfahrens beträgt das molare Verhältnis von p-Toluidin zu Wasserstoff 1:0,1 bis 1:5, vorzugsweise 1:1 bis 1:3,5, besonders bevorzugt 1:1 bis 1:3.

In einer weiteren Ausführungsform kann der bei der Reaktion z. B. durch die Zersetzung von Methanol entstehende Wasserstoff im Gemisch mit den weiteren Prozessgasen in einem Gaskreislauf in den Reaktor zurückgeführt werden.

In einer weiteren bevorzugten Ausführungsform des erfindungsgemäßen Verfahrens wird dieses bei einer Temperatur von 200 bis 300 °C durchgeführt, bevorzugt 210 bis 270 °C, besonders bevorzugt 220 bis 250 °C.

Ein weiterer Gegenstand der vorliegenden Erfindung ist ein Verfahren zur Herstellung eines Cu-, Zn- und Al-haltigen Katalysators durch Zugabe von wässriger Natriumcarbonatlösung sowie eines Gemisches aus wässriger Kupfernitrat- und Zinknitratlösung zu einer wässrigen Aluminiumoxidsuspension, unter Beibehaltung eines pH-Wertes von vorzugsweise 7,5 - 8,5, besonders bevorzugt 7,9 - 8,1, bei einer Temperatur von 60 - 80 °C, bevorzugt bei 65 - 75 °C, wobei das Gemisch aus wässriger Kupfernitrat- und Zinknitratlösung vor der Zugabe auf einen pH-Wert von < 1, vorzugsweise einen pH-Wert von 0,4 - 0,6 (bei 20 °C) eingestellt wird, die resultierende Cu-, Zn- und Al-haltige Suspension anschließend gewaschen, danach getrocknet, der daraus erhaltene Feststoff kalziniert und vorzugsweise tablettiert wird.

Die Einstellung des pH-Wertes auf < 1, vorzugsweise einen pH-Wert von 0,4 - 0,6 erfolgt dabei vorzugsweise mit Salpetersäure.

Die Einsatzmengen an Aluminiumoxidsuspension, Zinknitratlösung sowie Kupfernitratlösung hängen von der gewünschten Zusammensetzung des Cu-, Zn- und Al- haltigen Katalysators ab.

Nach Beendigung der Fällung wird die Suspension vorzugsweise zunächst über ein Querstromfilter aufkonzentriert und dann mit entionisiertem Wasser kontinuierlich über das Querstromfilter gewaschen.

In einer bevorzugten Ausführungsform der Erfindung wird die entstandene Suspension anschließend filtriert, gewaschen und der Feststoff im Trockenschrank getrocknet und vorzugsweise anschließend gesiebt. Alternativ kann der Feststoff auch durch Sprühtrocknung getrocknet werden. Das erhaltene Pulver wird vorzugsweise in einem Drehrohrofen bei 270 - 550 °C kalziniert, anschließend mit Graphit versetzt und mit einer Tablettenpresse zu Formkörpern verarbeitet.

Der nach dem erfindungsgemäßen Verfahren hergestellte Katalysator wird vorzugsweise zur Herstellung von N-Methyl-p-toluidin eingesetzt.

Gegenstand der vorliegenden Erfindung ist zudem der nach dem erfindungsgemäßen Verfahren hergestellte Katalysator mit einer spezifischen Kupferoberfläche von 1,4 - 10,2 m²/g, gemessen nach DIN66131-3.

Gegenstand der vorliegenden Erfindung ist zudem der nach dem erfindungsgemäßen Verfahren hergestellte Katalysator, mit einer durchschnittlichen Kupferpartikelgröße von mehr als 30 nm, bevorzugt 35 - 100 nm, besonders bevorzugt 35 - 75 nm, gemessen nach DIN66131-3.

Der Katalysator weist dabei vorzugsweise vor der Vorbehandlung die folgende Zusammensetzung auf und enthält zumindest in folgenden Anteilen
Cu: 45,5 - 68,0 Gew.%, vorzugsweise 47,0 - 64,1 Gew.%, besonders bevorzugt 47,5 - 60,5 Gew.%,
Zn: 12,3 - 22,5 Gew.%, vorzugsweise 14,3 - 21,5 Gew.%, besonders bevorzugt 15,2 - 19,5 Gew.%,
Al: 1,9 - 4,1 Gew.%, vorzugsweise 2,2 - 3,8 Gew.%, besonders bevorzugt 2,5 - 3,6 Gew.%,
sowie 7,8 - 28,2 Gew.% Sauerstoff, vorzugsweise 13,6 - 25,1 Gew.%, besonders bevorzugt 16,2 - 24,5 Gew.% Sauerstoff und gegebenenfalls 0 - 10 Gew.% Kohlenstoff enthält, wobei Cu, Zn, Al, Sauerstoff und gegebenenfalls Kohlenstoff mindestens 99 Gew.%, vorzugsweise 99,5 Gew.% der Gesamtmenge des Katalysators umfassen.

Ein weiterer Gegenstand der vorliegenden Erfindung ist N-Methyl-p-toluidin mit einem p-Toluidin-Gehalt > 0,0001 bis < 10 Gew.%, vorzugsweise > 0,001 bis < 1 Gew.%, welches vorzugsweise nach dem erfindungsgemäßen Verfahren hergestellt wurde.

Vorzugsweise enthält das erfindungsgemäße N-Methyl-p-toluidin neben dem p-Toluidin in Anteilen von > 0,0001 bis < 10 Gew.%, vorzugsweise > 0,001 bis < 1Gew.%, auch N,N-Dimethyl-p-toluidin in Anteilen von vorzugsweise > 0,0001 bis < 5 Gew.%.

N-Methyl-p-toluidin mit einem p-Toluidin-Gehalt > 0,0001 bis < 10 Gew.%, gegebenenfalls in Anwesenheit von N,N-Dimethyl-p-toluidin, zeichnet sich in Kombination mit einem Standard Flugbenzin Basefuel, durch einen Freezepoint von kleiner -58 °C, gemäß ASTM D23866 aus und ist daher hervorragend als Additiv für die Verwendung in bleifreiem Flugbenzin (AVGAS) gemäß ASTM D910 geeignet.

Um die für AVGAS benötigten Regelungen hinsichtlich Dampfdruck und Siedepunkten zu erfüllen, besitzen die für Flugbenzin Basefuel üblicherweise verwendeten Kohlenwasserstoffe eine Kohlenstoffnummer in der Größenordnung von vier (Butan) bis zehn mit einer vorwiegenden Häufigkeit von acht (Isooctan).

Darüber hinaus enthalten die Kohlenwasserstoff-Komponenten eines Standard Flugbenzin Basefuels üblicherweise isoparafinische (Butan, Pentan, Hexan, Heptan und Octan Isomeren) und aromatische Kohlenwasserstoffe (Benzol, Toluol, Ethylbenzol, Xylol Isomere, Trimethylbenzol, Cumol und Naphthalin).

Standard Flugbenzin Basefuel ist beispielsweise beschrieben in Chevron Aviation Fuels Technical Review 2M CBRES GIDC 5723 10/06 MS-9891 (10/06).

Ein weiterer Gegenstand der vorliegenden Erfindung ist die Verwendung des erfindungsgemäßen N-Methyl-p-toluidins sowie des nach dem erfindungsgemäßen Verfahren hergestellten N-Methyl-p-toluidins als Additiv für Benzine, vorzugsweise Flugbenzin.

Der Rahmen der Erfindung erfasst alle oben stehenden und im Folgenden aufgeführten allgemeinen oder in Vorzugsbereichen erwähnten Definitionen, Indizes, Parameter und Erläuterungen untereinander, also auch zwischen den jeweiligen Bereichen und Vorzugsbereichen in beliebiger Kombination.

Die nachfolgenden Beispiele dienen der Erläuterung der Erfindung, ohne dabei limitierend zu wirken.

### Beispiele

### Beispiel 1:

Herstellung des erfindungsgemäßen Katalysators:

### Vorbereitung:

### Vorlage 1:

18773 g Kupfernitratlösung mit 14,6 % Cu und 5522 g Zinknitratlösung mit 15,9 % Zn wurden mit destilliertem Wasser auf 22400 ml aufgefüllt. Anschließend wurde mit HNO₃ auf einen pH-Wert von 0,5 gestellt.

### Vorlage 2:

8959 g Natriumcarbonat wurden in 31038 g Wasser aufgelöst

### Durchführung:

Der Inhalt des Fällkessels, bestehend aus 40000 g entionisiertem Wasser, wurde mit einem Thermostaten auf 70 °C aufgeheizt. Die Vorlagen 1 und 2 wurden ebenfalls auf 70 °C aufgeheizt. Unter Rühren wurden 273,6 g Aluminiumoxid in den Fällkessel gegeben. Das Gemisch aus Kupfernitrat- und Zinknitratlösung aus Vorlage 1 wurde mit einer Membranpumpe kontinuierlich (190 - 220 ml/min) in den Fällkessel gepumpt, wobei eine zweite Membranpumpe die Natriumcarbonatlösung aus Vorlage 2 zupumpte, so dass während der Fällung der pH-Wert bei 7,9 - 8 gehalten wurde.
Die Suspension wurde anschließend am Querstromfilter aufkonzentriert und mit entionisiertem Wasser auf eine Leitfähigkeit < 100 µS gewaschen.

Die Suspension wurde über eine Porzellannutsche abgesaugt. Der Filterkuchen wurde im Trockenschrank bei 110 °C getrocknet und durch ein 0,8 mm Sieb gedrückt. Das Produkt wurde im Drehrohrofen bei 450 °C kalziniert.

4505 g des kalzinierten Produkts wurden mit 225 g Graphit auf einer Tablettenpresse (Typ Korsch XL 100) zu 5 x 5 mm Tabletten verarbeitet.

### Physikalische Eigenschaften:

Schüttgewicht: 1746 g/l,
Ø Seitenbruchhärte: 168 N,
BET-Oberfläche: 65,4 m²/g, bestimmt nach ISO9277,
Porenvolumen: 0,163 cm³/g,
Glühverlust (1 h, 900°C): 10,7 %,
Spezifische Cu-Oberfläche: 5,8 m²/g, gemessen nach DIN 66131-3,
Durchschnittliche Kupferpartikelgröße: 54,8 nm, gemessen nach DIN 66131-3.
Die Zusammensetzung des Katalysators ergab die folgenden Werte:
Cu: 51,0 Gew.%, Zn: 16,3 Gew.%, Al 2,7 Gew.%, O: 23,8 Gew.%, die sich nach Aufheizen auf 900 °C und einer Haltezeit von 1 h wie folgt verändert haben:
   Cu: 57,0 Gew.%, Zn: 18,3 Gew.%, Al: 2,9 Gew.%, O: 21,4 Gew.%

### Beispiel 2:

Austestung des erfindungsgemäßen Katalysators: 81 ml des nach Beispiel 1 hergestellten Katalysators (Siebfraktion 1,6 - 3,15 mm) wurden in ein Reaktionsrohr mit einem Innendurchmesser von 32 mm und einer Länge von 180 mm eingefüllt, in dem sich zusätzlich ein Innenrohr mit Thermoelement befand. Das Reaktionsrohr wurde durch eine elektrische Beheizung auf 275 °C gehalten, wobei zunächst der Katalysator mit einem Gemisch aus Stickstoff und Wasserstoff vorbehandelt wurde. Anschließend wurde kontinuierlich ein Reaktionsgemisch aus 40,4 g/h p-Toluidin, 24,1 g/h Methanol und 25,4 l/h Wasserstoff dosiert. Das Reaktionsprodukt wurde kondensiert und gaschromatographisch analysiert.
Die Zusammensetzung des Gemisches betrug nach Abzug von Wasser und Methanol, gemessen nach 300 Stunden:
p-Toluidin: 0,48 Gew.%
N-Methyl-p-toluidin: 96,27 Gew.%
N,N-Dimethyl-p-toluidin: 3,25 Gew.%
und nach 600 h:
   p-Toluidin: 0,59 Gew.%
   N-Methyl-p-toluidin: 97,67 Gew.%
   N,N-Dimethyl-p-toluidin: 1,74 Gew.%
   Selbst nach 600 Stunden Laufzeit war keine signifikante Desaktivierung des Katalysators zu erkennen.

### Vergleichsbeispiel 1:

Herstellung des Katalysators analog zu US-B2580284:
209,5 g Aluminiumoxid wurden mit 113 ml einer Tränklösung bestehend aus 94,33 g Calciumnitrat*4H₂O und 48,7 g Kupfer(II)-nitrat*3H₂O, gelöst in 34 g Wasser, versetzt. Nach der Tränkung wurde das Material im Warmlufttrockner bei 120 °C getrocknet und anschließend 4 Stunden bei 450 °C kalziniert. Es wurden 243,9 g Katalysator mit Metallanteilen von 5,17 Gew.% Cu und 6,46 Gew.% Ca auf Aluminiumoxid erhalten.

### Austestung des Katalysators:

81 ml des nach Vergleichsbeispiel 1 hergestellten Katalysators wurden analog zu Beispiel 1 für die Alkylierung von p-Toluidin eingesetzt.
Die typische Zusammensetzung des Gemisches betrug nach Abzug von Wasser und Methanol, gemessen nach 300 Stunden:
p-Toluidin: 33,02 Gew.%
N-Methyl-p-toluidin: 66,70 Gew.%
N,N-Dimethyl-p-toluidin: 0,28 Gew.%.

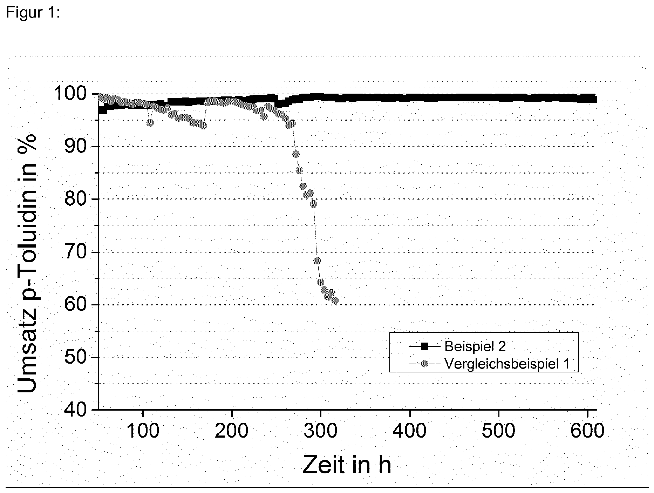

Der direkte Vergleich der beiden Katalysatoren ist in Figur 1 dargestellt. Es zeigt sich deutlich, dass der nach dem Stand der Technik eingesetzte Katalysator eine Standzeit von weniger als 300 Stunden aufweist, während der erfindungsgemäße Katalysator auch nach 600 Stunden keine Deaktivierung zeigt.

## Patentansprüche

1. Verfahren zur Herstellung von N-Methyl-p-toluidin durch Umsetzung von p-Toluidin mit Methanol, **dadurch gekennzeichnet, dass** mindestens ein Katalysator eingesetzt wird, der zumindest in folgenden Anteilen
Cu: 45,5 - 68,0 Gew.%, vorzugsweise 47,0 - 64,1 Gew.%, besonders bevorzugt 47,5 - 60,5 Gew.%,
Zn: 12,3 - 22,5 Gew.%, vorzugsweise 14,3 - 21,5 Gew.%, besonders bevorzugt 15,2 - 19,5 Gew.%,
Al: 1,9 - 4,1 Gew.%, vorzugsweise 2,2 - 3,8 Gew.%, besonders bevorzugt 2,5 - 3,6 Gew.%,
sowie 7,8 - 28,2 Gew.% Sauerstoff, vorzugsweise 13,6 - 25,1 Gew.%, besonders bevorzugt 16,2 - 24,5 Gew.% Sauerstoff und gegebenenfalls 0 - 10 Gew.% Kohlenstoff enthält, wobei Cu, Zn, Al, Sauerstoff und gegebenenfalls Kohlenstoff mindestens 99 Gew.%, vorzugsweise 99,5 Gew.% der Gesamtmenge des Katalysators umfassen.

2. Verfahren nach Anspruch 1, **dadurch gekennzeichnet, dass** der Katalysator durch Zufuhr von Wasserstoff, vorzugsweise in Gegenwart eines Inertgases, bei Temperaturen von mindestens 150 °C vorbehandelt wird.

3. Verfahren nach Anspruch 2, **dadurch gekennzeichnet, dass** die Vorbehandlung des Katalysators mit Wasserstoff vor dem Einsatz in den Reaktor oder im Reaktor selber erfolgen kann.

4. Verfahren nach einem der Ansprüche 1 bis 3, **dadurch gekennzeichnet, dass** die Belastung des Katalysators 0,1 bis 1,5, vorzugsweise 0,3 bis 1, besonders bevorzugt 0,4 bis 0,7 kg p-Toluidin pro kg Katalysator und Stunde beträgt.

5. Verfahren nach einem der Ansprüche 1 bis 4, **dadurch gekennzeichnet, dass** das molare Verhältnis von p-Toluidin zu Methanol 1:0,7 bis 1:5, vorzugsweise 1:1 bis 1:3,5, besonders bevorzugt 1:1,5 bis 1:2,5 beträgt.

6. Verfahren nach einem der Ansprüche 1 bis 5, **dadurch gekennzeichnet, dass** das molare Verhältnis von p-Toluidin zu Wasserstoff 1:0,1 bis 1:5, vorzugsweise 1:0,5 bis 1:4, besonders bevorzugt 1:1 bis 1:3 beträgt.

7. Verfahren nach einem der Ansprüche 1 bis 6, **dadurch gekennzeichnet, dass** das Verfahren bei einer Temperatur von 200 bis 300 °C durchgeführt wird.

8. Verfahren zur Herstellung eines Cu-, Zn- und Al-haltigen Katalysators durch Zugabe von wässriger Natriumcarbonatlösung sowie eines Gemisches aus wässriger Kupfernitrat- und Zinknitratlösung zu einer wässrigen Aluminiumoxidsuspension, unter Beibehaltung eines pH-Wertes von vorzugsweise 7,5 - 8,5, besonders bevorzugt 7,9 - 8,1, bei einer Temperatur von 60 - 80 °C, bevorzugt bei 65 - 75 °C, wobei das Gemisch aus wässriger Kupfernitrat- und Zinknitratlösung vor der Zugabe auf einen pH-Wert von < 1, vorzugsweise einen pH-Wert von 0,4 - 0,6 (bei 20 °C) eingestellt wird, die resultierende Cu-, Zn- und Al-haltige Suspension anschließend gewaschen, danach getrocknet, der daraus erhaltene Feststoff kalziniert und vorzugsweise tablettiert wird.

9. Katalysator, erhältlich durch ein Verfahren nach Anspruch 8, mit einer spezifischen Kupferoberfläche von 1,4 - 10,2 m²/g, gemessen nach DIN66131-3.

10. Katalysator nach Anspruch 9, mit einer durchschnittlichen Kupferpartikelgröße von mehr als 30 nm, bevorzugt 35 - 100 nm, besonders bevorzugt 35 - 75 nm, gemessen nach DIN66131-3.

11. N-Methyl-p-toluidin mit einem p-Toluidin-Gehalt > 0,0001 bis < 10 Gew.%, vorzugsweise > 0,001 bis < 1 Gew.%.

12. N-Methyl-p-toluidin mit einem p-Toluidin-Gehalt > 0,0001 bis < 10 Gew.%, vorzugsweise > 0,001 bis < 1 Gew.% und N,N-Dimethyl-p-toluidin in Anteilen von > 0,0001 bis < 5 Gew.%.

13. N-Methyl-p-toluidin, erhältlich nach einem Verfahren nach einem der Ansprüche 1 bis 7.

14. Verwendung des nach dem Verfahren nach einem der Ansprüche 1 bis 7 hergestellten N-Methyl-p-toluidins als Additiv für Benzine, bevorzugt Flugbenzine.

15. Verwendung des Katalysators nach Anspruch 9 oder 10 zur Herstellung von N-Methyl-p-toluidin.
